# EUROPEAN PATENT SPECIFICATION

(11) **EP 4 486 274 B1**
(45) Date of publication and mention of the grant of the patent: **23.09.2026**
(21) Application number: 23730195.7
(22) Date of filing: 24.02.2023
(51) Int. Cl.: A61F 13/00, A61F 13/02

(54) **APPARATUS AND SYSTEM FOR MANAGING TUBING AT A TISSUE SITE**
VORRICHTUNG UND SYSTEM ZUR VERWALTUNG VON SCHLÄUCHEN AN EINER GEWEBESTELLE
APPAREIL ET SYSTÈME DE GESTION DE TUBE AU NIVEAU D'UN SITE TISSULAIRE

(30) Priority: 01.03.2022 US 202263315327 P
(43) Date of publication of application: 08.01.2025
(73) Proprietor: Solventum Intellectual Properties Company, Eagan, MN 55121 (US)
(72) Inventor: HOWARD, Robert, Bracknell Berkshire RG12 8HT (GB); PRATT, Benjamin A., Bracknell Berkshire RG12 8HT (GB); NOLAN, Dominic, Bracknell Berkshire RG12 8HT (GB)
(74) Representative: Simmons & Simmons
(86) International application number: PCT/IB2023/051737
(87) International publication number: WO 2023/166393

(56) References cited:
- US-A1- 2020 197 228
- US-A1- 2020 276 383

## Description

### TECHNICAL FIELD

The invention set forth in the appended claims relates generally to tissue treatment systems and more particularly, to an apparatus for managing tubing at a tissue site.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.
US 2020/276383 A1 relates to infusion sets for a delivering a medication to a patient, and more particularly to a tube management device for an infusion set.

### BRIEF SUMMARY

The invention is defined by the independent claim. A selection of optional features of the invention is set out in the dependent claims.

Insofar as the term embodiment is used in the following, or features are presented as being optional, this should be interpreted in such a way that the only protection sought is that of the invention claimed (with due regard to Article 69 EPC and the protocol thereto).

References to "embodiment(s)" throughout the description which are not under the scope of the appended claims merely represent possible exemplary executions and are not part of the present invention.

### BRIEF DESCRIPTION OF THE DRAWINGS

Figure 1 is a block diagram of an example embodiment of a therapy system that can provide negative-pressure treatment in accordance with this specification;
Figure 2 is a perspective view of an example embodiment of the therapy system of Figure 1, illustrating additional details that maybe associated with some embodiments;
Figure 3A is a perspective view of an example embodiment of a dressing and a therapy device of Figure 2, illustrating additional details that may be associated with some embodiments;
Figure 3B is a perspective view of an example embodiment of a dressing interface of Figure 3A, illustrating additional details that may be associated with some embodiments;
Figure 3C is a cross-section view of the dressing interface of Figure 3B, illustrating additional details that may be associated with some embodiments;
Figure 4A is a perspective view of the dressing and the therapy device of Figure 3A in a closed position, illustrating additional details that may be associated with some embodiments;
Figure 4B is a perspective view of the dressing interface of Figure 3B in a closed position, illustrating additional details that may be associated with some embodiments;
Figure 4C is a cross-section view of the dressing interface of Figure 4B in the closed position, illustrating additional details that may be associated with some embodiments;
Figure 5 is a top, interior view of an example embodiment of a dressing interface of the therapy system of Figure 1, illustrating additional details that may be associated with some embodiments;
Figure 6A is a cross-section view of the dressing interface of Figure 5 applied to an example dressing and tissue site, illustrating additional details that may be associated with some embodiments;
Figure 6B is a section detail-view of a dressing of Figure 6A, illustrating additional details that may be associated with some embodiments;
Figure 7A is a perspective view of an example embodiment of a dressing and a therapy device of the therapy system of Figure 1 in a first position, illustrating additional details that may be associated with some embodiments;
Figure 7B is a perspective view of the dressing and the therapy device of Figure 7A in a second position, illustrating additional details that may be associated with some embodiments;
Figure 8 is a perspective view of another example embodiment of the therapy system of Figure 1, illustrating additional details that maybe associated with some embodiments;
Figure 9A is a top view of an inline coil of Figure 8, illustrating additional details that maybe associated with some embodiments;
Figure 9B is a perspective view of the inline coil of Figure 9A in an open position, illustrating additional details that maybe associated with some embodiments;
Figure 9C is a perspective view of the inline coil of Figure 9A in a closed position, illustrating additional details that maybe associated with some embodiments;
Figure 10A is a perspective view of another example of the inline coil of Figure 8 in an open position, illustrating additional details that maybe associated with some embodiments;
Figure 10B is a perspective view of another example of the inline coil of Figure 8 in a closed position, illustrating additional details that maybe associated with some embodiments;
Figure 11A is a top view of an optional offloading layer that can be associated with the inline coil of Figure 8, illustrating additional details that may be associated with some embodiments;
Figure 11B is a perspective view of the offloading layer associated with the inline coil of Figure 11A in an open position, illustrating additional details that may be associated with some embodiments;
Figure 11C is a perspective view of the offloading layer associated with the inline coil of Figure 11A in a closed position, illustrating additional details that may be associated with some embodiments;
Figure 12A is a perspective view of another example embodiment of the inline coil of Figure 8, illustrating additional details that may be associated with some embodiments;
Figure 12B is a top, interior view of the inline coil of Figure 12A, illustrating additional details that may be associated with some embodiments;
Figure 12C is a perspective cut-away view of the inline coil of Figure 12A, illustrating additional details that may be associated with some embodiments;
Figure 12D is a cross-section view of the inline coil of Figure 12A, illustrating additional details that may be associated with some embodiments;
Figure 13A is a perspective view of another example embodiment of the inline coil of Figure 8, illustrating additional details that may be associated with some embodiments;
Figure 13B is a cross-section view of the inline coil of Figure 13A in an open position, illustrating additional details that may be associated with some embodiments;
Figure 13C is another cross-section view of the inline coil of Figure 13A, illustrating additional details that may be associated with some embodiments;
Figure 13D is a perspective view of the inline coil of Figure 13A in a closed position, illustrating additional details that may be associated with some embodiments;
Figure 13E is a cross-section view of the inline coil of Figure 13D, illustrating additional details that may be associated with some embodiments;
Figure 14 is a perspective view of another example embodiment of the therapy system of Figure 1, illustrating additional details that maybe associated with some embodiments;
Figure 15A is a perspective view of an example tubing clamp of Figure 14, illustrating additional details that may be associated with some embodiments;
Figure 15B is a perspective view of the tubing clamp of Figure 15A, illustrating additional details that may be associated with some embodiments;
Figure 15C is another perspective view of the tubing clamp of Figure 15A, illustrating additional details that may be associated with some embodiments;
Figure 16A is a perspective view of another example embodiment of the tubing clamp of Figure 14, illustrating additional details that may be associated with some embodiments;
Figure 16B is a perspective view of the tubing clamp of Figure 16A, illustrating additional details that may be associated with some embodiments;
Figure 17 is a graph illustrating details that may be associated with some embodiments of the therapy system of Figure 1; and
Figure 18 is a graph illustrating additional details that may be associated with some embodiments of the therapy system of Figure 1.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

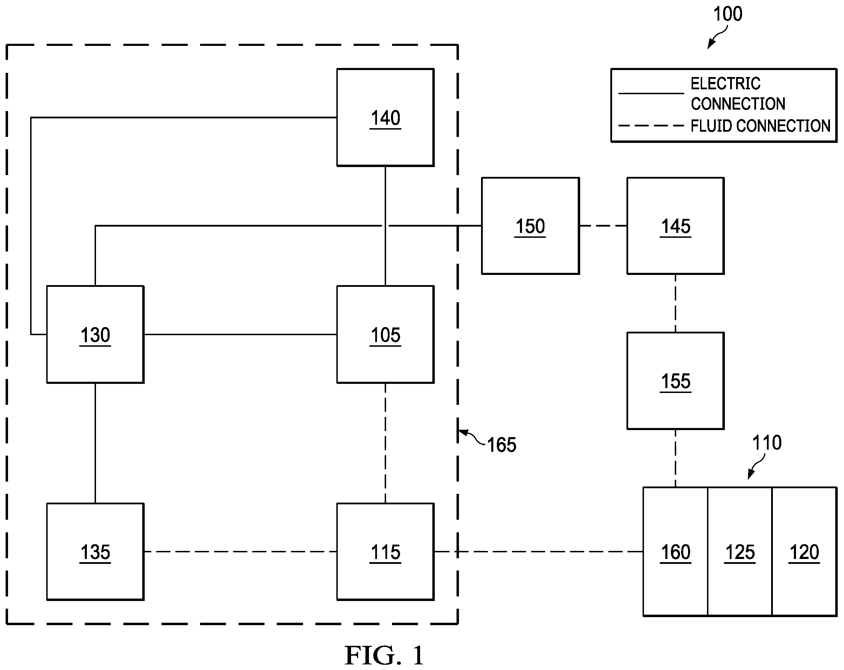

Figure 1 is a block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments. Additionally, the dressing 110 may also include a dressing interface 160 in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit 165.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. For example, the tensile strength of foam may be increased for instillation of topical treatment solutions. The 25% compression load deflection of the tissue interface 120 may be at least 0.35 pounds per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per square inch (1 pound per square inch = 4.882422 kgf/m²). In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The process of reducing pressure may be described illustratively herein as "delivering," "distributing," or "generating" negative pressure, for example. In general, exudate and other fluid flow toward lower pressure along a fluid path. Thus, the term "downstream" typically implies a location in a fluid path relatively closer to a source of negative pressure or further away from a source of positive pressure. Conversely, the term "upstream" implies a location relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed without limitation in some applications, such as by substituting a positive-pressure source for a negative-pressure source.

Negative pressure applied to the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

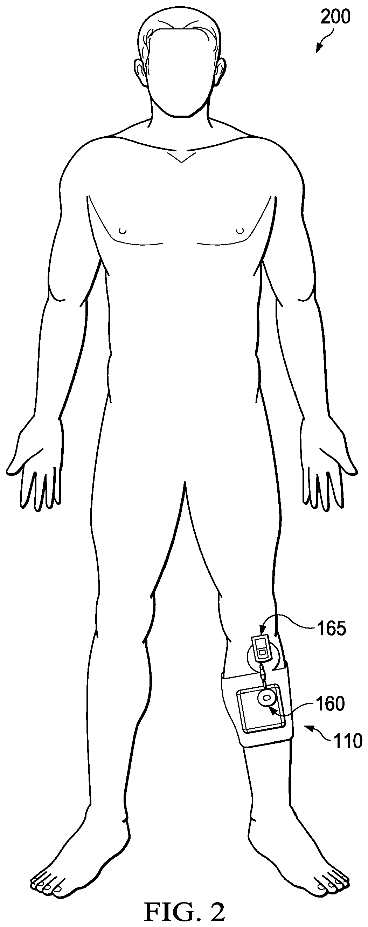

Figure 2 is a perspective view of an example embodiment of the therapy system 100 of Figure 1, illustrating additional details that maybe associated with some embodiments. In some embodiments, the therapy unit 165 and the dressing 110 may be positioned on epidermis of a patient 200. For example, the dressing interface 160 of the dressing 110 may be positioned adjacent a tissue site or wound on the patient 200 and the therapy unit 165 may be positioned on healthy epidermis of the patient 200 proximate the dressing 110.

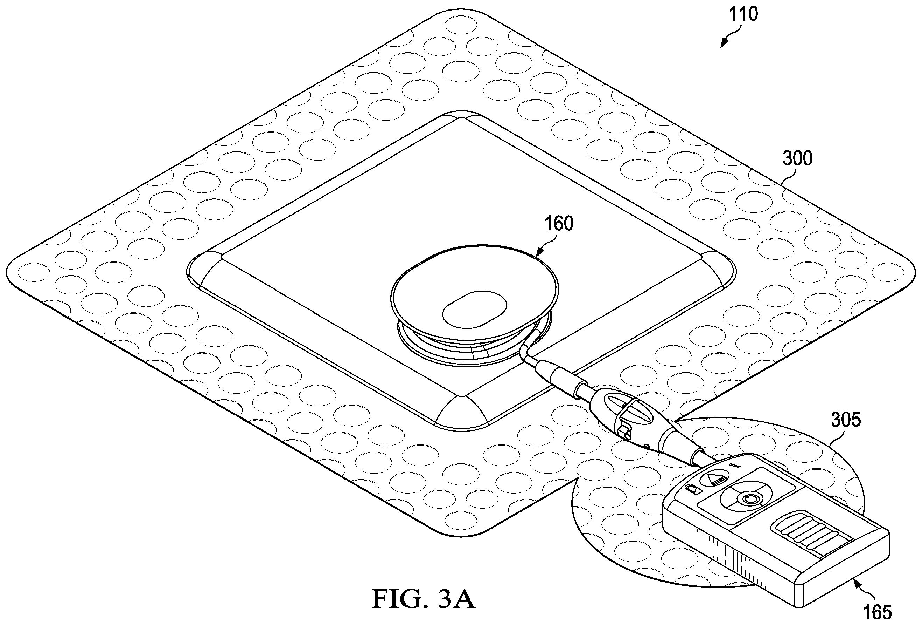

Figure 3A is a perspective view of the therapy unit 165 and the dressing interface 160 of the dressing 110. In some embodiments, the dressing interface 160 is coupled to a first film layer 300. The first film layer 300 may be configured to be coupled to a tissue site. For example, the first film layer 300 may be coupled to the tissue site by an adhesive. Additionally, the therapy unit 165 is coupled to a second film layer 305. The second film layer 305 may be configured to be coupled to the tissue site. For example, the second film layer 305 may also be coupled to the tissue site with an adhesive. In some embodiments, the second film layer 305 is coupled to the tissue site adjacent the first film layer 300. In some additional embodiments, the first film layer 300 and the second film layer 305 are attached and configured to be separable from each other.

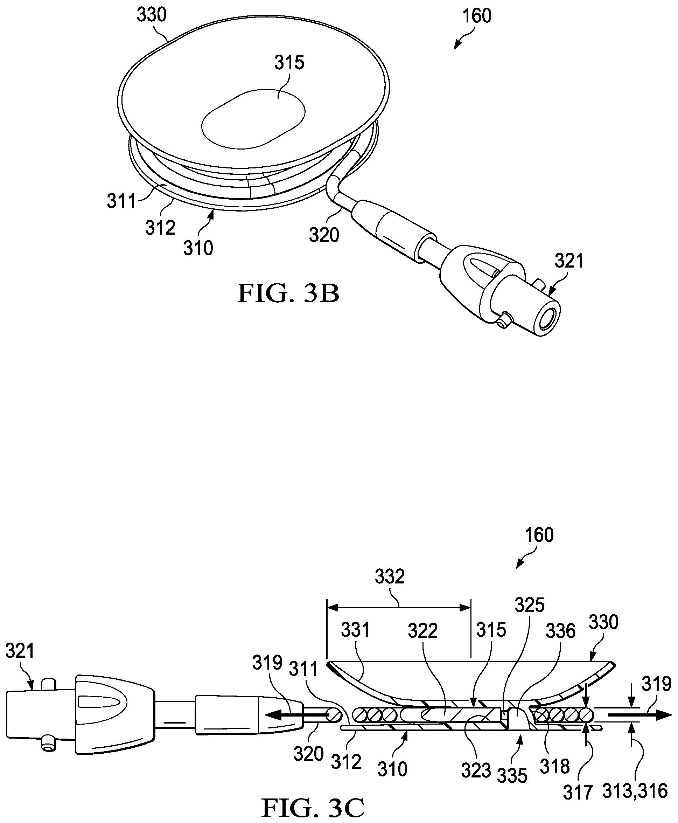

Referring to Figures 3B and 3C, the dressing interface 160 comprises a base 310 having a first side 311 and a second side 312. In some embodiments, a post member 315 extends outward from the first side 311 of the base 310. For example, the post member 315 may include a length 313 or a thickness extending from the first side 311 of the base. In some embodiments, the post member 315 comprises an ovular shape. In other embodiments, the post member 315 comprises a circular shape. In some additional embodiments, at least a portion of the base 310 may include a plurality of apertures or perforations. For example, the plurality of apertures or perforations may be disposed in the base 310 in an area surrounding the post member. In such embodiments, moisture and/or vapor may pass through the base 310 from the tissue site.

The post member 315 includes a winding surface 318 around an exterior surface of the post member 315, such as around the length 313 of the post member 315. In some embodiments, the winding surface 318 is configured to carry the conduit 320. For example, the conduit 320 may be configured to be coiled around the winding surface 318 of the post member 315. In some embodiments, the conduit 320 may be configured to be coiled around the winding surface 318 of the post member 315 in a single layer having a thickness 316 that does not exceed an outside diameter 317 of the conduit 320. For example, the conduit 320 may be coiled around the winding surface 318 such that each revolution of the conduit 320 around the winding surface 318 lies in the same plane 319. In such an example, the length 313 of the post member 315 may be substantially the same as the thickness 316 of the single layer of the conduit 320 and the outside diameter 317 of the conduit 320. The outside diameter 317 of the conduit 320 may be selected to suit a particular need.

In some embodiments, the dressing interface 160 includes a port 325 coupled to the post member 315. For example, the port 325 may be positioned in a recess 323 in the winding surface 318. The port 325 may be configured to fluidly couple the conduit 320 to the tissue site. For example, the conduit 320 may comprise a first end 321 configured to be fluidly coupled to a negative pressure source, such as the negative-pressure source 105, and a second end 322 configured to be fluidly coupled to the port 325. In some embodiments, the first end 321 of the conduit 320 may be configured to be coupled to the therapy unit 165. The port 325 may extend through the post member 315 and be fluidly coupled to the tissue site through an aperture 335 in the base 310. For example, the aperture 335 may extend from the first side 311 to the second side 312 of the base and be fluidly coupled to the port 325 through an internal chamber 336 in the post member 315. The internal chamber 336 may be in fluid communication between the port 325 and the aperture 335 such that the conduit 320 is configured to be fluidly coupled to a tissue site through the internal chamber 336 and the aperture 335.

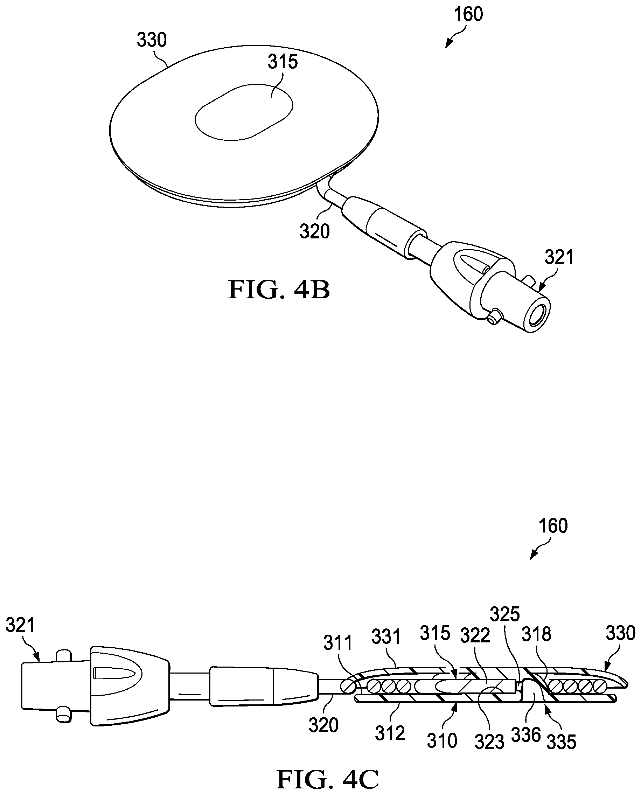

Additionally, the dressing interface 160 includes a top flange 330 coupled to the post member 315 opposite the base 310. The top flange 330 may include a deformable lip 331 having a radial length 332 extending transverse to the length 313 of the post member 315. The deformable lip 331 may be configured to move between an open position, as shown in Figures 3B and 3C, and a closed position, shown in Figures 4B-4C. In the open position, the deformable lip 331 of the top flange 330 may be configured to extend outward from the first side 311 of the base 310 to expose the winding surface 318.

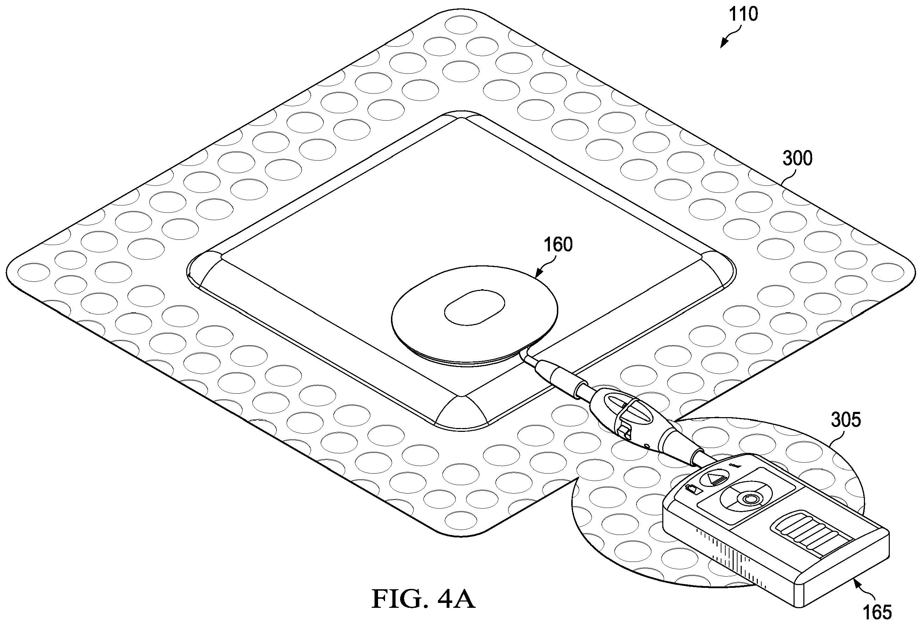

With reference to Figures 4A-4C, the deformable lip 331 of the top flange 330 may be positioned closer to the first side 311 of the base 310 in the closed position than in the open position. In such embodiments, the deformable lip 331 of the top flange 330 may be configured to extend inward toward the first side 311 of the base 310 in the closed position to hold the conduit 320 in place between the deformable lip 331 and the first side 311 of the base 310. The deformable lip 331 may be configured to extend over the base 310 in the closed position. In other embodiments, the deformable lip 331 of the top flange may be configured to remain in the closed position. For example, the conduit 320 may be coiled around the winding surface 318 while the deformable lip 331 is in the closed position. In such embodiments, the deformable lip 331 may comprise a flexible material, such as an elastomer, allowing the deformable lip 331 to at least partially flex when coiling the conduit 320. Additionally or alternatively, the deformable lip 331 may include at least one chamfer configured to guide the conduit 320 under the deformable lip 331 when being coiled around the winding surface 318.

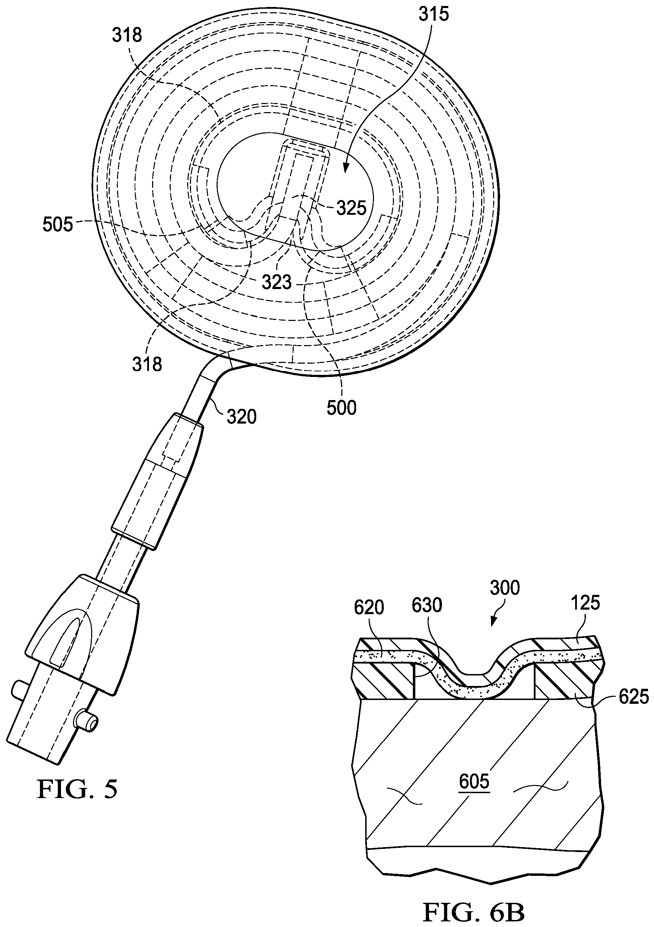

Figure 5 is a top, interior view of an exemplary embodiment of the dressing interface 160. In some embodiments, the winding surface 318 of the post member 315 may include at least one curved portion adjacent the port 325. For example, the winding surface 318 of the post member 315 may include a first curved portion 500 adjacent the port 325. Additionally or alternatively, the winding surface 318 of the post member 315 may include a second curved portion 505 adjacent the port 325 and opposite the first curved portion 500. The port 325 may be positioned between the first curved portion 500 and the second curved portion 505. For example, the port 325 may be positioned in the recess 323 between the first curved portion 500 and the second curved portion 505. In some embodiments, the first curved portion 500 and the second curved portion 505 comprise a radius corresponding to a bending radius of the conduit 320. Further, the first curved portion 500 and the second curved portion 505 may lie in the same plane 319 as shown in Figure 3C.

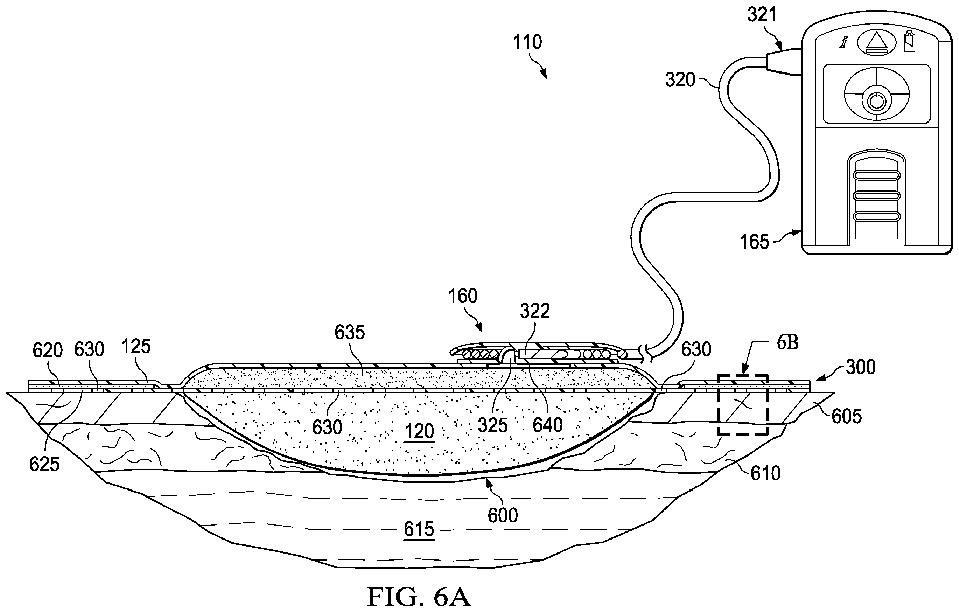

Figure 6A is a cross-section view of the dressing 110, illustrating additional details that may be associated with some embodiments. In operation, the tissue interface 120 may be placed within, over, on, or otherwise proximate to a tissue site, such as a tissue site 600. If the tissue site is a wound, for example, the tissue interface 120 may partially or completely fill the wound, or it may be placed over the wound. In the example embodiment of Figures 6A, the tissue site 600 extends through an epidermis 605, or generally skin, and a dermis 610 reaching into a hypodermis, or a subcutaneous tissue 615. In some examples, the tissue interface 120 may be omitted and the dressing 110 may be applied directly to or over the tissue site 600.

The dressing 110 may be positioned over the tissue site 600. For example, the first film layer 300 may be positioned over the tissue interface 120 and coupled to undamaged epidermis 605 adjacent the tissue site 600. With reference to Figure 6B, the first film layer 300 may comprise the cover 125 and a sealing layer 625. The sealing layer 625 may comprise or consist essentially of a soft, pliable material suitable for providing a fluid seal with a tissue site, and may have a substantially flat surface. For example, the sealing layer 625 may comprise, without limitation, a silicone gel, a soft silicone, hydrocolloid, hydrogel, polyurethane gel, polyolefin gel, hydrogenated styrenic copolymer gel, a foamed gel, a soft closed cell foam such as polyurethanes and polyolefins coated with an adhesive, polyurethane, polyolefin, or hydrogenated styrenic copolymers. In some embodiments, the sealing layer 625 may have a thickness between about 200 microns (µm) and about 1000 microns (µm). In some embodiments, the sealing layer 625 may have a hardness between about 5 Shore OO and about 80 Shore OO. Further, the sealing layer 625 may be comprised of hydrophobic or hydrophilic materials. In some embodiments, the sealing layer 625 may be a hydrophobic-coated material. For example, the sealing layer 625 may be formed by coating a spaced material, such as, for example, woven, nonwoven, molded, or extruded mesh with a hydrophobic material. The hydrophobic material for the coating may be a soft silicone, for example.

In some embodiments, a plurality of apertures 630 may be disposed in the sealing layer 625. The apertures 630 may be formed by cutting or by application of local RF or ultrasonic energy, for example, or by other suitable techniques for forming an opening. The apertures 630 may have a uniform distribution pattern, or may be randomly distributed on the sealing layer 625. The apertures 630 in the sealing layer 625 may have many shapes, including circles, squares, stars, ovals, polygons, slits, complex curves, rectilinear shapes, triangles, for example, or may have some combination of such shapes.

Each of the apertures 630 may have uniform or similar geometric properties. For example, in some embodiments, each of the apertures 630 may be circular apertures, having substantially the same diameter. In some embodiments, the diameter of each of the apertures 630 may be between about 1 millimeter to about 50 millimeters. In other embodiments, the diameter of each of the apertures 630 may be between about 1 millimeter to about 20 millimeters.

In other embodiments, geometric properties of the apertures 630 may vary. For example, the diameter of the apertures 630 may vary depending on the position of the apertures 630 in the sealing layer 625. In some embodiments, the diameter of the apertures 630 in a periphery of the sealing layer 625 may be larger than the diameter of the apertures 630 in an interior portion of the sealing layer 625.

In some embodiments, the first film layer 300 may further include an attachment device, such as an adhesive 620. The adhesive 620 may be, for example, a medically-acceptable, pressure-sensitive adhesive that extends about a periphery, a portion, or the entire cover 125. In some embodiments, for example, the adhesive 620 may be an acrylic adhesive having a coating weight between 25-65 grams per square meter (g.s.m.). Thicker adhesives, or combinations of adhesives, may be applied in some embodiments to improve the seal and reduce leaks. The adhesive 620 may be a layer having substantially the same shape as the periphery of the sealing layer 625. In some embodiments, such a layer of the adhesive 620 may be continuous or discontinuous. Discontinuities in the adhesive 620 may be provided by apertures or holes (not shown) in the adhesive 620. The apertures or holes in the adhesive 620 may be formed after application of the adhesive 620 or by coating the adhesive 620 in patterns on a carrier layer, such as, for example, a side of the cover 125. Apertures or holes in the adhesive 620 may also be sized to enhance the MVTR of the dressing 110 in some example embodiments.

Referring again to Figure 6A, the first film layer 300 may also include a manifold 635 positioned between the cover 125 and the sealing layer 625. In some embodiments, the manifold 635 provides a means for collecting or distributing fluid across the tissue interface 120 under pressure. For example, the manifold 635 may be adapted to receive negative pressure from the negative-pressure source 105 of the therapy unit 165 and distribute negative pressure through the apertures 630 across the tissue interface 120, which may have the effect of collecting fluid from across the tissue site 600 and drawing the fluid toward the therapy unit 165. In some embodiments, the fluid path may be reversed or a secondary fluid path may be provided to facilitate delivering fluid, such as from a source of instillation solution, across the tissue interface 120.

In some embodiments, the dressing interface 160 is configured to be coupled to the first film layer 300. For example, the dressing interface 160 may be coupled to the cover 125 of the first film layer 300. In some embodiments, the dressing interface 160 is configured to fluidly couple the therapy unit 165 to the tissue site 600. For example, the second end 322 of the conduit 320 may be fluidly coupled to the therapy unit 165 and the first end 321 of the conduit 320 may be fluidly coupled to the port 325 of the dressing interface 160. The dressing interface 160 may fluidly couple the conduit 320 to the tissue site 600 through the port 325, the aperture 335 in the base 310, an aperture 640 in the cover 125, and one or more of the apertures 630 in the sealing layer 625.

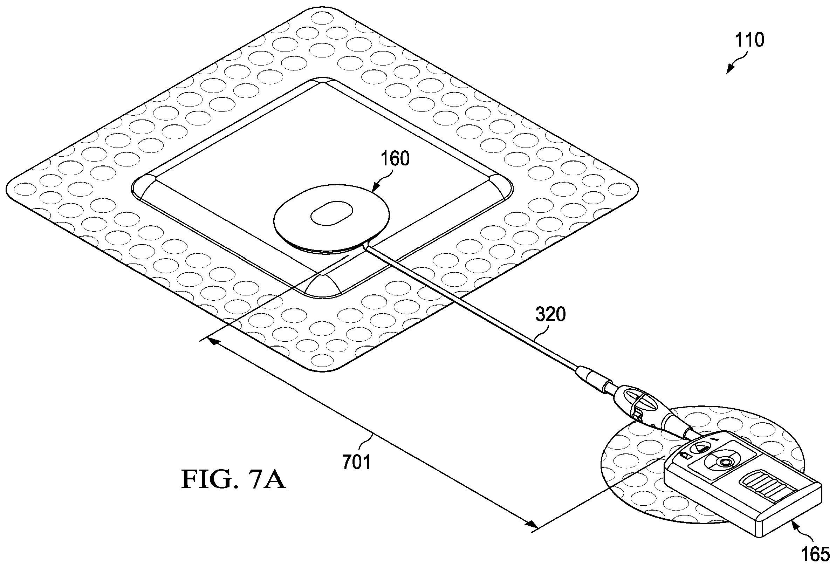

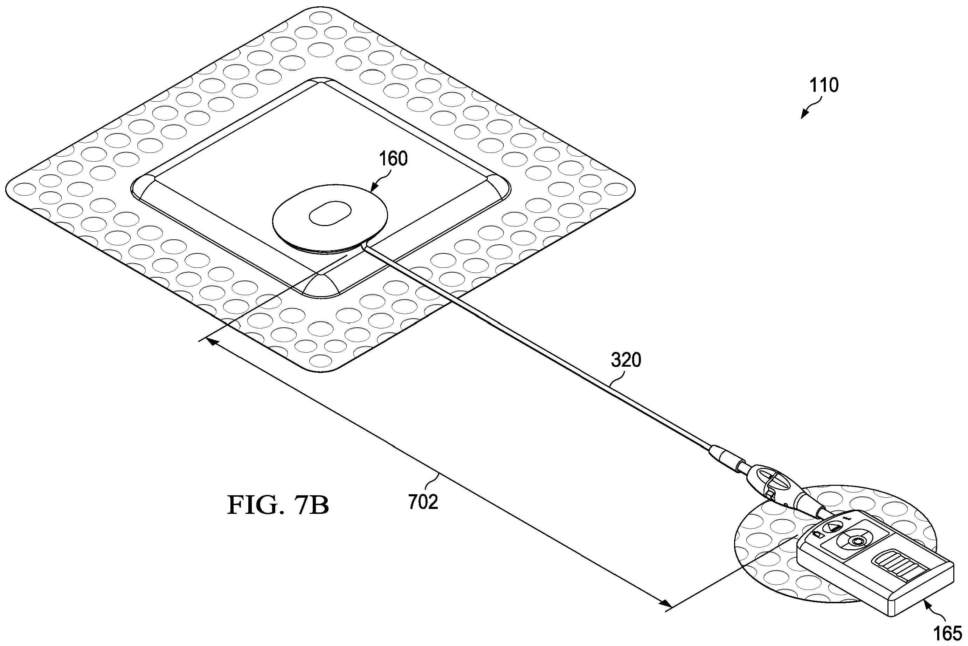

With reference to Figures 7A and 7B, the conduit 320 comprises a length. In some embodiments, the length of the conduit 320 may be between about 5 centimeters and about 30 centimeters. In other embodiments, the length of the conduit 320 may be between about 30 centimeters and about 200 centimeters. In still other embodiments, the length of the conduit 320 may be about 30 centimeters. The conduit 320 may be coiled or wrapped around the dressing interface 160 to reach a desired length. For example, as shown in Figure 7A, the conduit 320 may be wrapped around the dressing interface 160 to achieve a first distance 701 between the dressing interface 160 and the therapy unit 165. Alternatively, as shown in Figure 7B, the conduit 320 may be wrapped around the dressing interface 160 to achieve a second distance 702 between the dressing interface 160 and the therapy unit 165. The first distance 701 and the second distance 702 may be different. For example, the second distance 702 may be greater than the first distance 701. The first distance 701 and the second distance 702 may be selected by a clinician to suit a particular need, such as patient comfort or ease of accessibility.

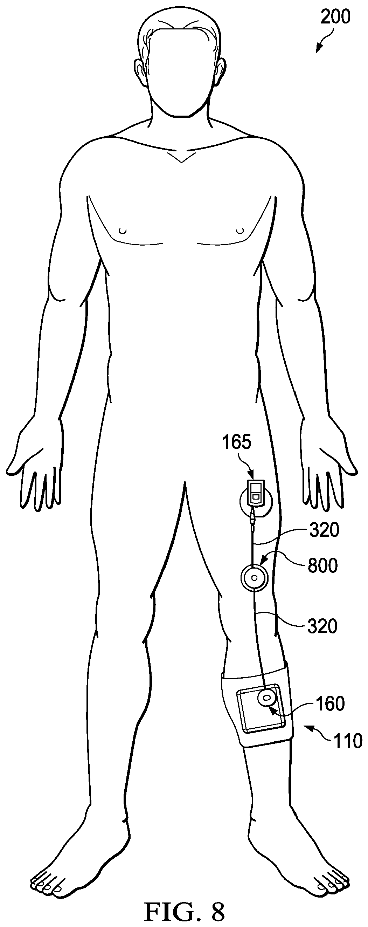

Figure 8 is a perspective view of another example embodiment of the therapy system 100 of Figure 1, illustrating additional details that maybe associated with some embodiments. In some embodiments, the therapy system 100 may include an inline coil 800 in addition to the dressing 110, the dressing interface 160, and the therapy unit 165. For example, the inline coil 800 may be configured to be coupled to the patient 200 between the dressing 110 and the therapy unit 165.

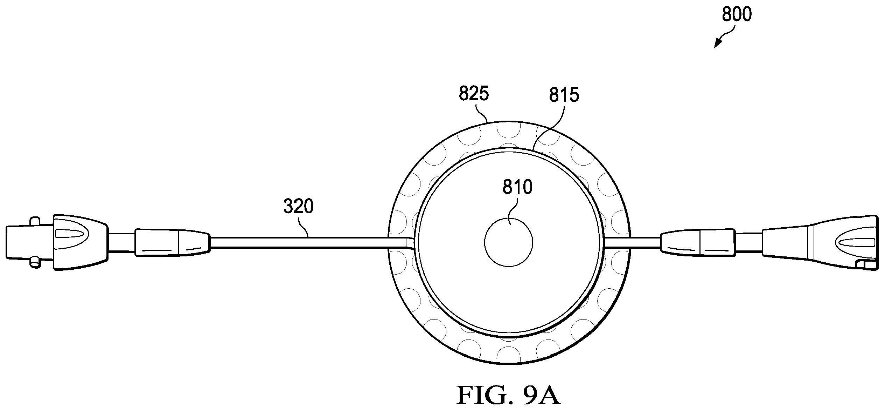

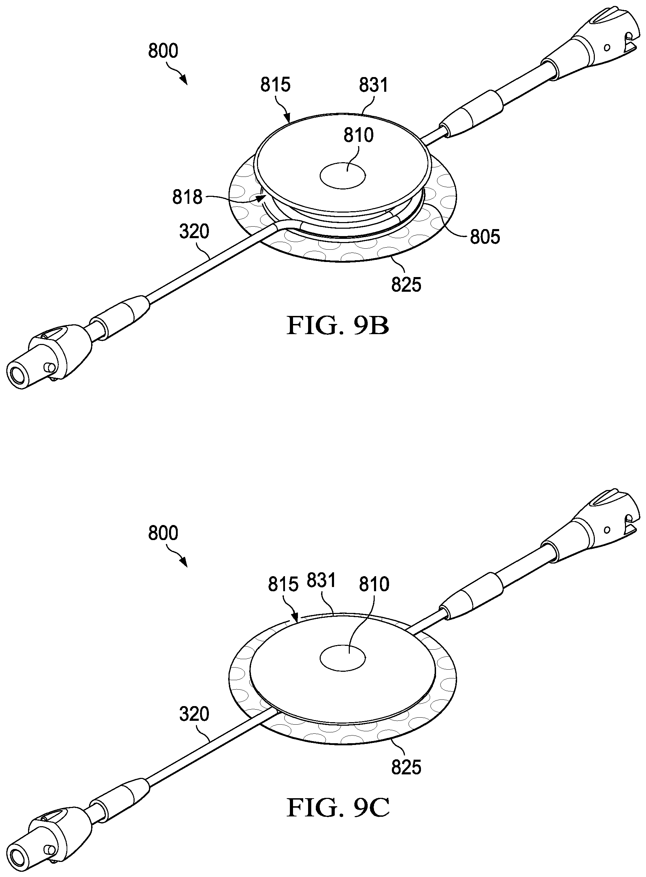

With reference to Figures 9A-9C, the inline coil 800 may be similar or analogous to the dressing interface 160. For example, the inline coil 800 may include a base 805, a post member 810 extending from a first side of the base 805, and a top flange 815 coupled to the post member 810 opposite the base 805. The top flange 815 may include a deformable lip 831 configured to move between an open position, as shown in Figure 9B, and a closed position, as shown in Figure 9C. The deformable lip 831 of the top flange 815 may also be configured to operate the same way as the deformable lip 331 of the top flange 330, as discussed above with respect to Figures 3A-3C.

The post member 810 of the inline coil 800 may include a winding surface 818 configured to carry a conduit, such as the conduit 320. The winding surface 818 may be analogous to the previously described winding surface 318 illustrated in Figures 4C and 5. In some embodiments, the winding surface 818 of the post member 810 may comprise a circular or ovular shape. The conduit 320 may be configured to be coiled around the winding surface 818 of the post member 810 in a single layer having a thickness that does not exceed an outside diameter of the conduit 320. For example, the conduit 320 may be coiled around the winding surface such that each revolution of the conduit 320 around the winding surface 818 lies in the same plane. In some embodiments, the conduit 320 may be coiled and uncoiled about the winding surface 818 of the post member 810 from one or both ends of the conduit 320.

In some embodiments, a third film layer 825 may be configured to couple the inline coil 800 to epidermis of the patient 200. For example, the third film layer 825 may be coupled to the second side of the base 805, opposite the top flange 815. The third film layer 825 may be the same as or similar to the first film layer 300 discussed above with respect to Figures 6A and 6B.

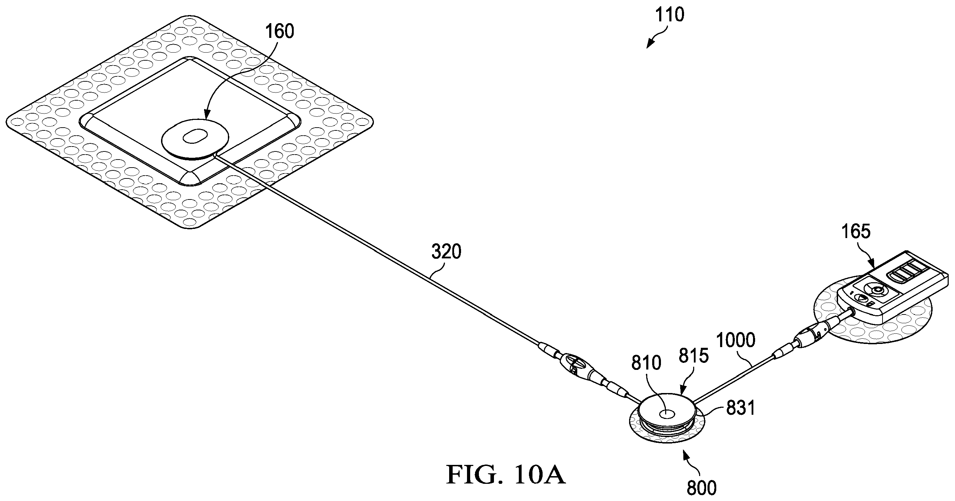

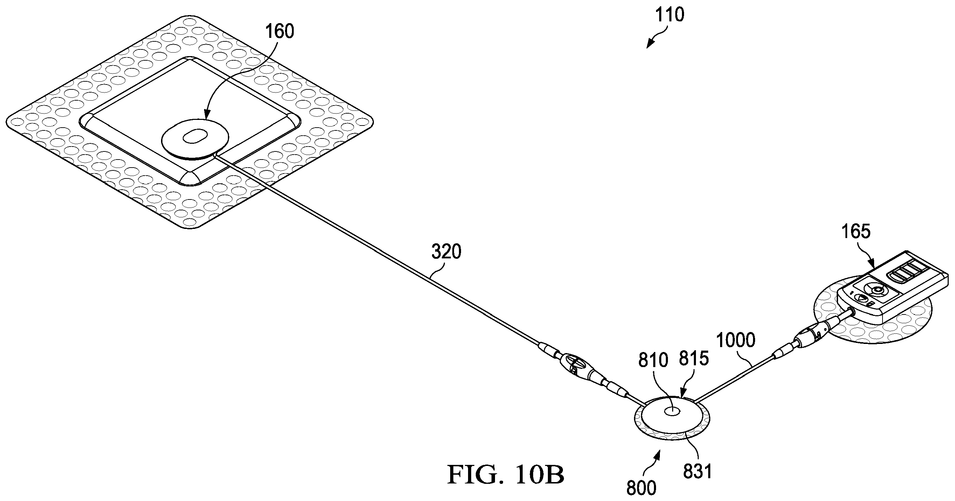

With reference to Figures 10A and 10B, the inline coil 800 may be used with the dressing interface 160 of the dressing 110 and the therapy unit 165. In some embodiments, a second conduit 1000 may be at least partially coiled around the winding surface 818 of the post member 810 of the inline coil 800. For example, the second conduit 1000 may be coupled between the conduit 320 and the therapy unit 165. The inline coil 800 may allow for the therapy unit 165 to be positioned further from a tissue site while still managing the tubing, such as the conduit 320 and/or the second conduit 1000. Additionally or alternatively, the inline coil 800 may be used to change the direction of the tubing, as shown in Figures 10A and 10B. For example, the inline coil 800 may be positioned adjacent an articulating joint. In some embodiments, the conduit 320 or the second conduit 1000 may be coiled around the post member 810 of the inline coil 800 until a desired length and a desired direction of the conduit 320 or the second conduit 1000 is achieved. The deformable lip 831 of the top flange 815 may be in the open position, as shown in Figure 10A. The deformable lip 831 of the top flange 815 may then be placed in the closed position to secure the conduit 320 or the second conduit 1000 at the desired length and the desired direction, as shown in Figure 10B.

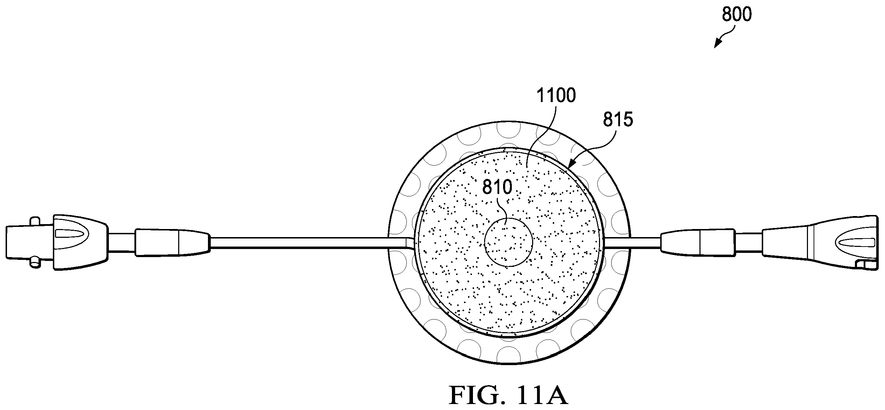

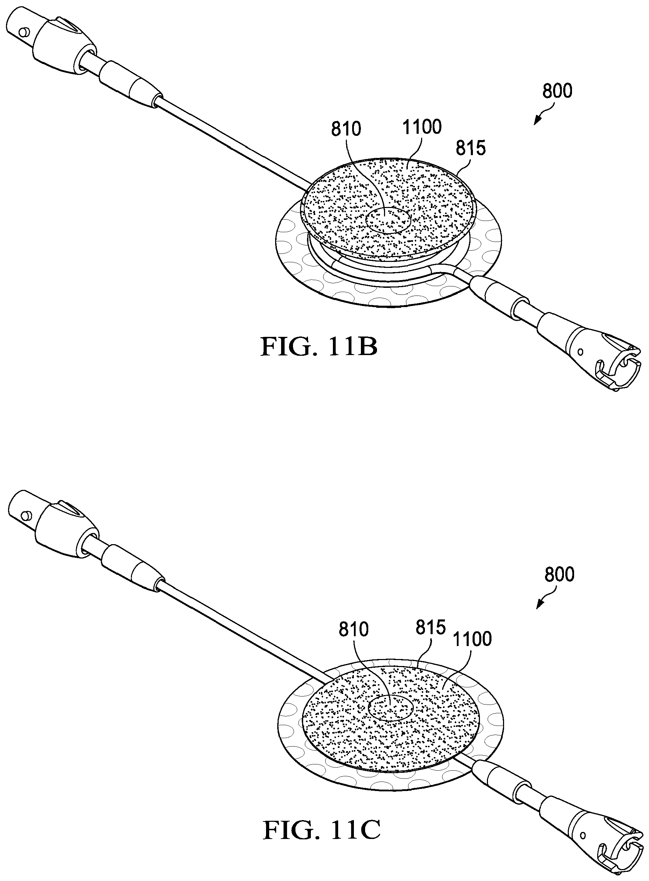

With reference to Figures 11A-11C, the inline coil 800 may include an optional offloading layer 1100 in some embodiments. For example, the offloading layer 1100 may be configured to be coupled to the top flange 815 and a top surface of the post member 810. The offloading layer 1100 may be configured to reduce pressure points on a patient's epidermis resulting from the tubing, such as the conduit 320 and/or the second conduit 1000 from coiling over itself. In some embodiments, the offloading layer 1100 comprises a foam. In some additional embodiments, the offloading layer 1100 may be coupled to the top flange 330 and a top surface of the post member 315 of the dressing interface 160.

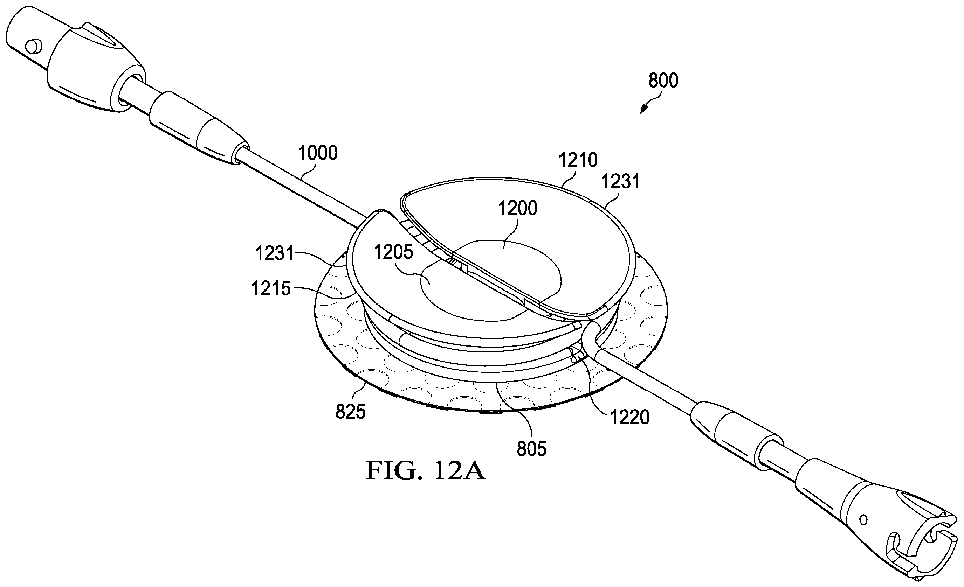

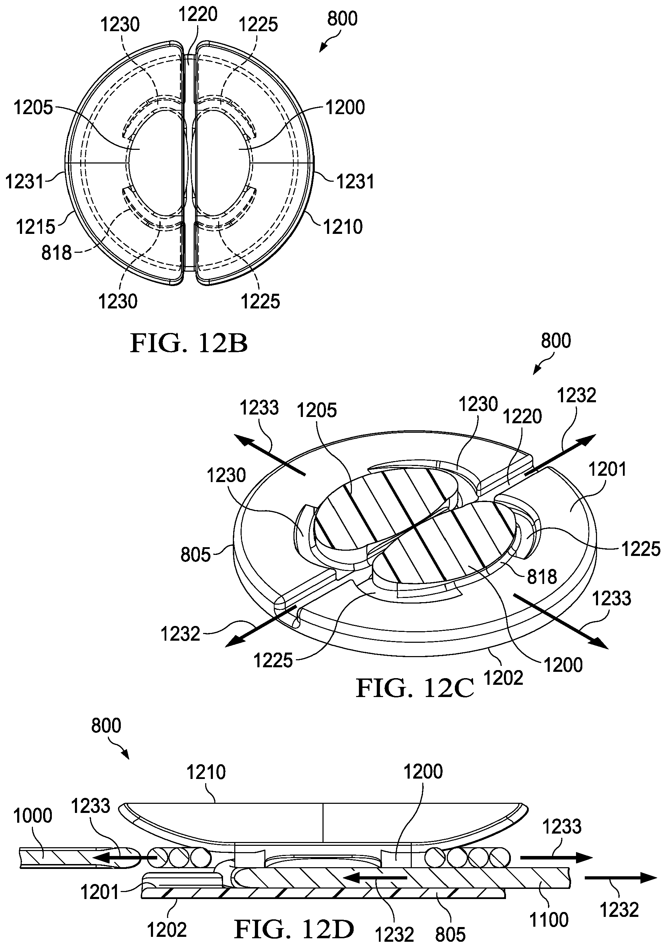

Figure 12A is a perspective view of another embodiment of the inline coil 800, illustrating additional details that may be associated with some embodiments. In some embodiments, the inline coil 800 may include a first post 1200 and a second post 1205 adjacent the first post 1200. As shown in Figure 12C, the first post 1200 and the second post 1205 may extend from a first side 1201 of the base 805. In some embodiments, the first post 1200 and the second post 1205 may comprise a semicircular shape or ovular. In other embodiments, the first post 1200 and the second post 1205 may comprise a circular shape.

In some embodiments, the top flange 815 comprises a first portion 1210 and a second portion 1215. The first portion 1210 may be configured to be coupled to the first post 1200 and the second portion 1215 may be configured to be coupled to the second post 1205. The first portion 1210 and the second portion 1215 may each include a deformable lip 1231 configured to move between an open position, as shown in Figures 12A and 12D, and a closed position. In the closed position, the deformable lip 1231 of each of the first portion 1210 and the second portion 1215 may be configured to be positioned closer to the base 805 than in the open position.

With reference to Figures 12A-12D, a longitudinal recess 1220 extends between the first post 1200 and the second post 1205 in a first plane 1232 adjacent the first side 1201 of the base 805. The longitudinal recess 1220 may also extend between the first portion 1210 and the second portion 1215 of the top flange 815. In some embodiments, the base 805 also includes a first taper 1225 and a second taper 1230. The first taper 1225 may be configured to extend from the longitudinal recess 1220 and around the first post 1200 to a second plane 1233 above the first plane 1232, and the second taper 1230 may be configured to extend from the longitudinal recess 1220 and around the second post 1205 to the second plane 1233.

In some embodiments, the second conduit 1000 is configured to be inserted into the longitudinal recess 1220 in the first plane 1232. The second conduit 1000 may be configured to extend from the longitudinal recess 1220 into the second plane 1233 via the first taper 1225 or the second taper 1230. The second conduit 1000 may also be configured to be coiled around the first post 1200 and the second post 1205 such that each revolution of the second conduit 1000 around an exterior surface of the first post 1200 and the second post 1205 lies in the second plane 1233. In other embodiments, the conduit 320 may be inserted into the longitudinal recess 1220 and coiled around the first post 1200 and the second post 1205 via the first taper 1225 or the second taper 1230 as described for the second conduit 1000.

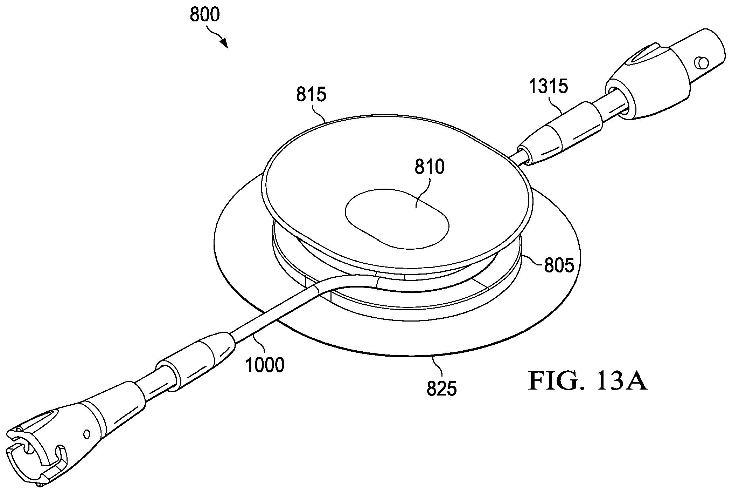

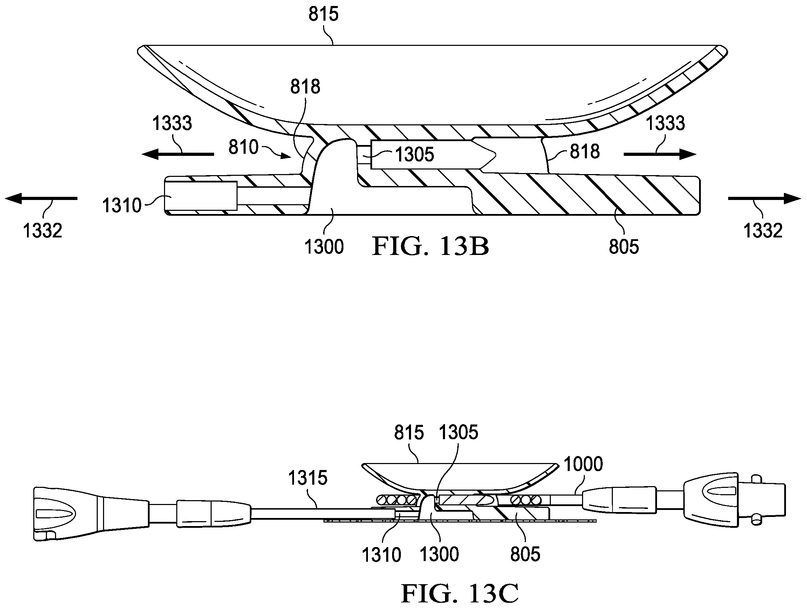

Referring to Figures 13A-13E, another embodiment of the inline coil 800 is shown, illustrating additional details that may be associated with some embodiments. In some embodiments, the inline coil 800 may include an internal cavity 1300, disposed in the base 805 and the post member 810. A first port 1305 may be coupled to a side of the post member 810 and a second port 1310 may be coupled to a side of the base 805 opposite the first port 1305. In some embodiments, the second port 1310 may be in a first plane 1332 and the first port 1305 may be in a second plane 1333 above the first plane 1332. Additionally, the first port 1305 and the second port 1310 may be configured to be fluidly coupled to the internal cavity 1300. For example, the first port 1305 and the second port 1310 may be fluidly coupled via the internal cavity 1300.

In some embodiments, a base conduit 1315 is coupled to the second port 1310 and configured to fluidly couple the inline coil 800 to the dressing 110. For example, the base conduit 1315 may be configured to be fluidly coupled to the dressing 110 via the conduit 320 of Figures 10A-10B. In other embodiments, the base conduit 1315 may be configured to be fluidly coupled to the dressing 110 directly.

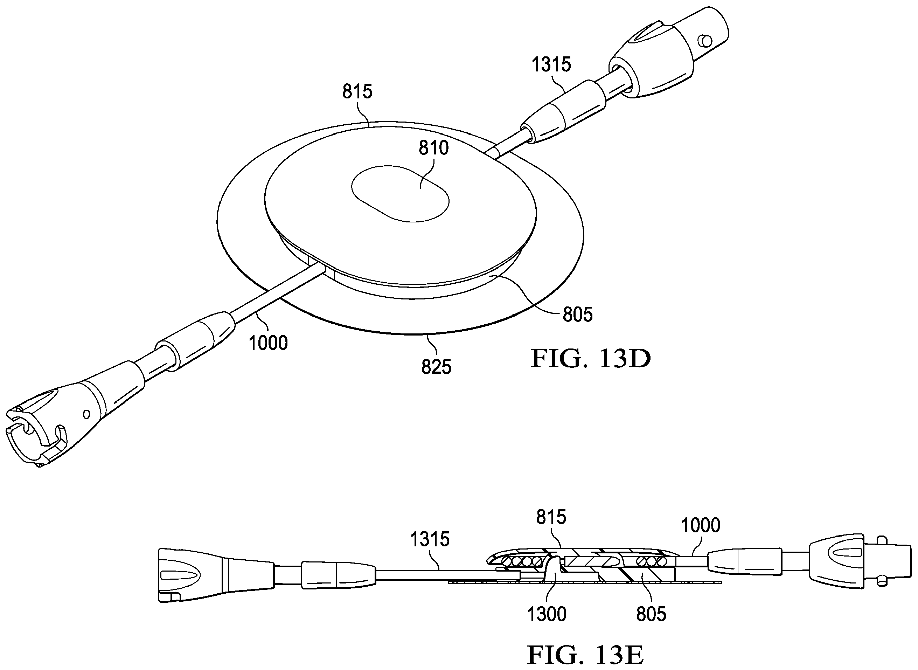

In some embodiments, the second conduit 1000 is fluidly coupled to the first port 1305. As discussed above with respect to Figures 10A and 10B, the second conduit 1000 may be configured to be coiled around the winding surface 818 of the post member 810. For example, the second conduit 1000 may be coiled around the post member 810 when the top flange 815 is in the open position, as shown in Figures 13A-13C, until a desired length of the second conduit 1000 is reached. Once the desired length of the second conduit 1000 is reached, the top flange 815 may be moved into the closed position, as shown in Figures 13D and 13E, to secure the second conduit 1000. The second conduit 1000 may be configured to be coiled around the post member 810 such that each revolution of the second conduit 1000 around the post member 810 lies in a single layer and in the same plane.

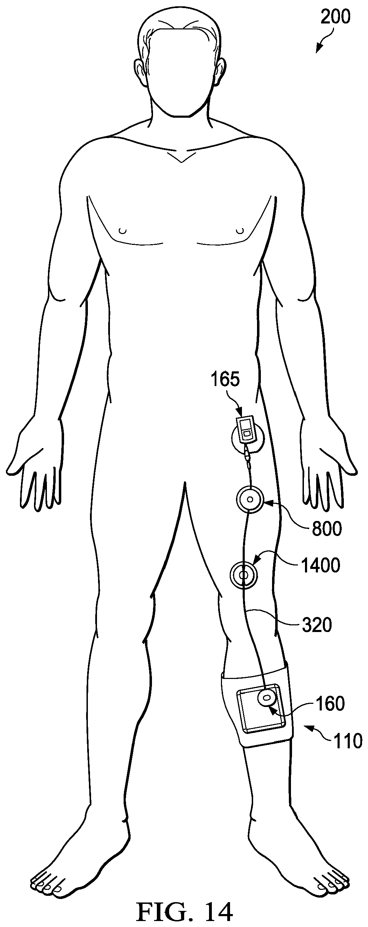

Figure 14 is a perspective view of another example embodiment of the therapy system 100 of Figure 1, illustrating additional details that maybe associated with some embodiments. In some embodiments, the therapy system 100 may include a tubing clamp 1400 in addition to one or more of the dressing 110, the dressing interface 160, the inline coil 800, and the therapy unit 165. For example, the tubing clamp 1400 may be configured to be coupled to the patient 200 between the dressing 110 and the therapy unit 165.

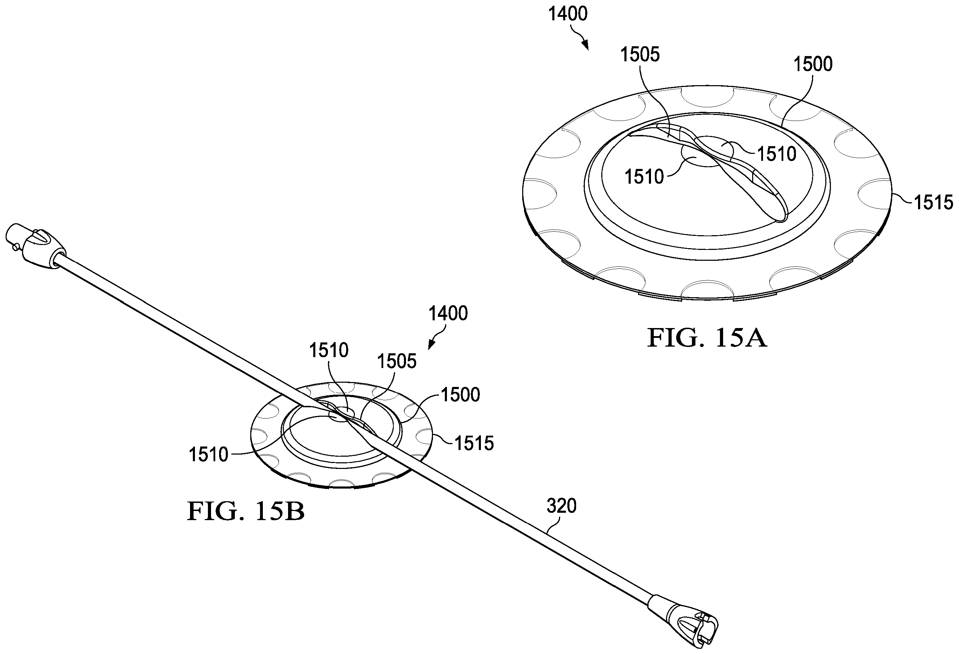

Figure 15A is a perspective view of the tubing clamp 1400, illustrating additional details that may be associated with some embodiments. In some embodiments, the tubing clamp 1400 includes a pad member 1500 and a slot 1505 disposed in the pad member 1500. As shown in Figure 15B, the slot 1505 may be configured to receive a conduit, such as the conduit 320. In other embodiments, the slot 1505 may be configured to receive the second conduit 1000. In still other embodiments, the slot 1505 may be configured to receive another conduit configured to be fluidly coupled to the conduit 320 and/or the second conduit 1000.

In some embodiments, the slot 1505 is configured to extend across a top surface of the pad member 1500. Additionally, at least a portion of the top surface of the pad member 1500 may taper away from a center of the slot 1505 to an outer edge of the pad member 1500 to form gripping members 1510. The gripping members 1510 may be formed on either side of the slot 1505 and are configured to grip the conduit 320. For example, the gripping members 1510 are configured to secure the conduit 320 in the slot 1505.

In some embodiments, the tubing clamp 1400 may be configured to be coupled proximate to a tissue site of the patient 200, as shown in Figure 14. For example, a fourth film layer 1515 may be configured to couple the tubing clamp 1400 to the tissue site. The fourth film layer 1515 may be similar or analogous to the first film layer 300 discussed above with reference to Figures 6A-6B.

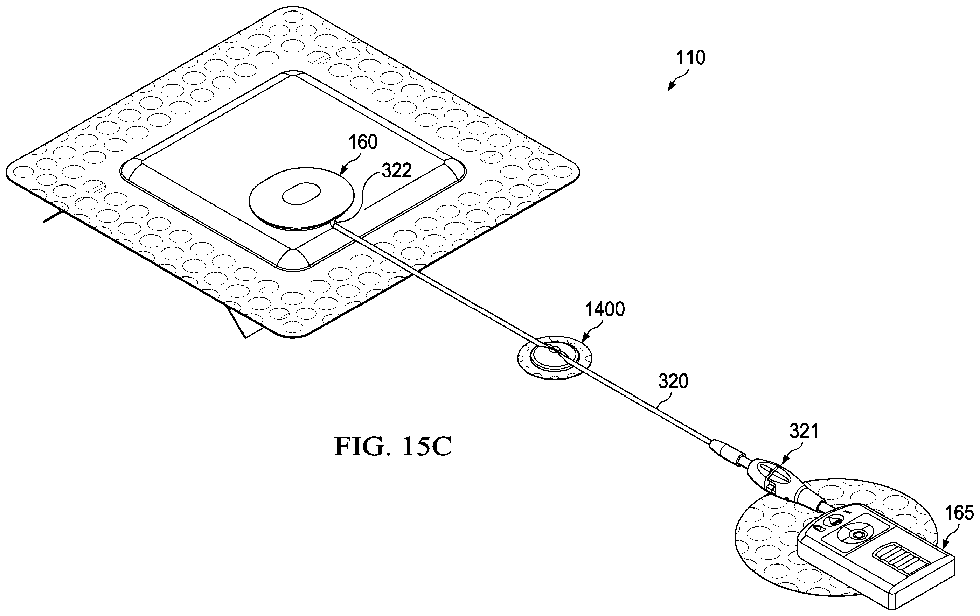

With reference to Figure 15C, the first end 321 of the conduit 320 may be configured to be fluidly coupled to the therapy unit 165 and the second end 322 of the conduit 320 may be configured to be fluidly coupled to the dressing interface 160 of the dressing 110. In some embodiments, the conduit 320 is coupled to one or more of the tubing clamps 1400 to prevent the conduit 320 from hanging loose off the patient 200.

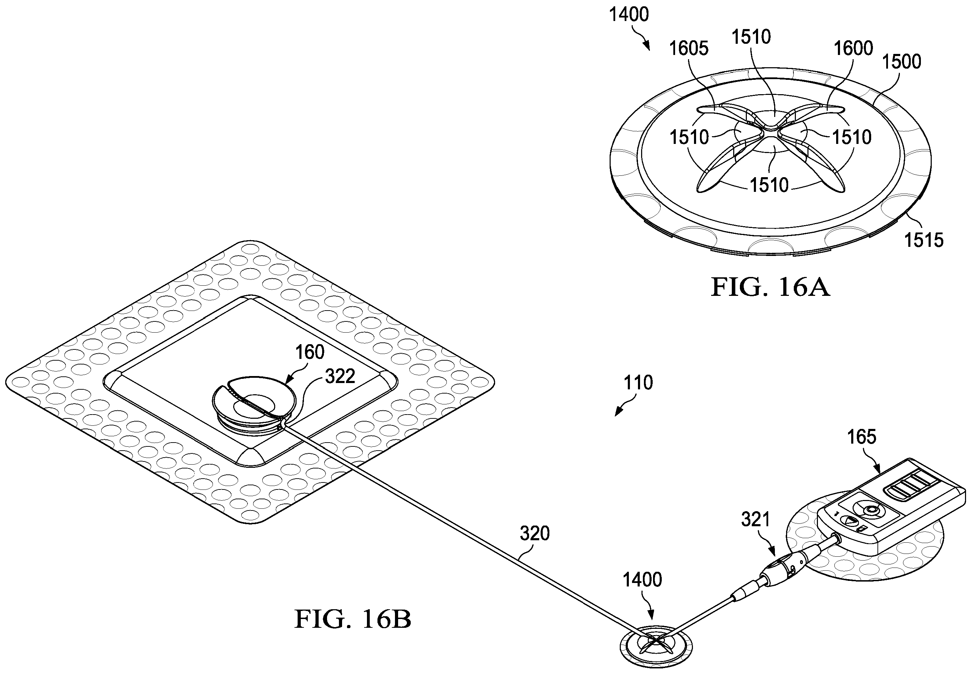

Figure 16A is a perspective view of another embodiment of the tubing clamp 1400, illustrating additional details that may be associated with some embodiments. In some embodiments, the tubing clamp 1400 includes a first slot 1600 and a second slot 1605 disposed in the pad member 1500. The first slot 1600 and the second slot 1605 intersect and extend radially outward from a center of the pad member. For example, an angle between the first slot 1600 and the second slot 1605 may be about 90° or less. The first slot 1600 and the second slot 1605 may also form the gripping member 1510 adjacent the center of the pad member 1500.

With reference to Figure 16B, the first slot 1600 and the second slot 1605 of the tubing clamp 1400 may be configured to receive the conduit 320. The gripping members 1510 may be configured to secure the conduit 320 within the tubing clamp 1400. In some embodiments, the first slot 1600 and the second slot 1605 may be configured to allow the conduit 320 to extend straight though the tubing clamp 1400 or turn at an angle. For example, the conduit 320 may extend through a portion of the first slot 1600 and the second slot 1605 to turn at an angle, as shown in Figure 16B. In other embodiments, the conduit 320 may be inserted through only the first slot 1600 or the second slot 1605 to extend straight through the tubing clamp 1400.

Similar to the tubing clamp 1400 discussed with respect to Figure 15C, the first end 321 of the conduit 320 may be configured to be fluidly coupled to the therapy unit 165 and the second end 322 of the conduit 320 may be configured to be fluidly coupled to the dressing interface 160 of the dressing 110. In some embodiments, the conduit 320 is coupled to one or more of the tubing clamps 1400 to prevent the conduit 320 from hanging loose off the patient 200.

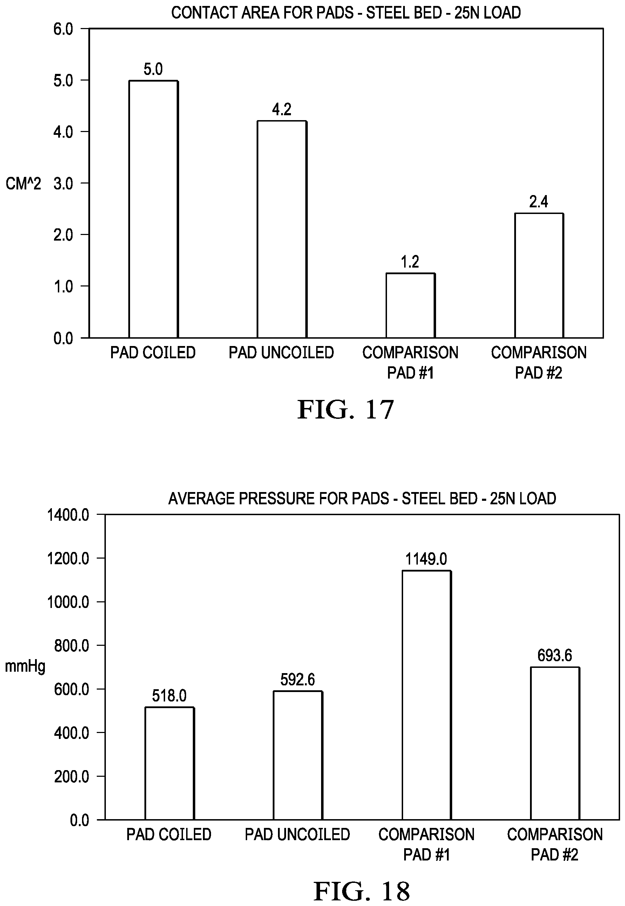

Figure 17 is a chart illustrating the contact area, or pressure points, exerted by various pads for managing tubing. For example, testing was performed on the dressing interface 160 with tubing coiled around the dressing interface 160 and tubing uncoiled around the dressing interface 160. Testing was conducted by which a load of 25N was applied evenly across a top surface of the dressing interface 160 and conventional comparison pads, which did not include any features for coiling or managing tubing as described herein.

The data demonstrates that the coiled and uncoiled pads have a greater contact area, which results in less pressure points and more even pressure distribution. With respect to the dressing interface 160 coiled (Pad Coiled), the contact area was 5 cm². With respect to the dressing interface 160 uncoiled (Pad Uncoiled), the contact area was 4.2 cm². With respect to Comparison Pad 1, the contact area was 1.2 cm². With respect to Comparison Pad 2, the contact area was 2.4 cm².

Figure 18 is a chart illustrating the average pressure exerted by various pads for managing tubing. For example, testing was performed on the dressing interface 160 with tubing coiled around the dressing interface 160 and tubing uncoiled around the dressing interface 160. Testing was conducted by which a load of 25N was applied evenly across a top surface of the dressing interface 160 and the conventional comparison pads referenced above.

The data demonstrates that the coiled and uncoiled pads exert less pressure than the comparison pads. The data also demonstrates that the average pressure exerted by each of the pads is inversely proportional to the contact area shown in Figure 17. With respect to the dressing interface 160 coiled (Pad Coiled), a pressure of 518 mmHG was exerted. With respect to the dressing interface 160 uncoiled (Pad Uncoiled), a pressure of 592.6 mmHG was exerted. With respect to Comparison Pad 1, a pressure of 1149 mmHg was exerted. With respect to Comparison Pad 2, a pressure of 693.6 was exerted.

The systems, apparatuses, and methods described herein may provide significant advantages. For example, tubing may be coiled and uncoiled about the dressing interface 160. Additionally, the flexible nature of the top flange 330 of the dressing interface 160 allows the top flange to be popped open or popped closed to hold the tubing in place. The dressing interface 160 improves patient comfort by reducing pressure points exerted on the body of a patient by the dressing interface 160. For example, the dressing interface 160 prevents the tubing from overlapping itself as it is coiled, which reduces possible pressure points. Additionally or alternatively, an offloading layer, such as a foam layer, may be coupled to the dressing interface to reduce the pressure points.

The therapy system 100 also provides an inline coil 800 for managing tubing. A patient or caregiver may select where to position the inline coil 800 relative to the tissue site based on desired patient mobility and comfort. For example, a patient or caregiver can position the inline coil directly next to a tissue site. In such embodiments, the tubing may remain coiled around the inline coil 800. Alternatively, a patient or caregiver may position the inline coil elsewhere on the patient's body. In such embodiments, the coil may be uncoiled to the desired length. Additionally, additional tubing may be coupled together to customize the length of the tubing.

Moreover, the tubing clamp 1400 can be used in conjunction with the dressing interface 160 and/or the inline coil 800. The tubing claim 1400 may secure the tubing closer to the patient's body and prevent the tubing from hanging loosely, which reduces possible trip hazards.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or eliminated in various configurations for purposes of sale, manufacture, assembly, or use. For example, in some configurations the dressing 110, the container 115, or both may be eliminated or separated from other components for manufacture or sale. In other example configurations, the controller 130 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art.

## Claims

1. An apparatus for managing tubing, comprising:
a base (310) having a first side (311) and a second side (312);
a post member (315) extending from the first side (311) of the base (310), the post member (315) including a winding surface (318) that carries a conduit (320); and
a top flange (330) coupled to the post member (315) opposite the base (310), the top flange (330) including a deformable lip (331) which moves between an open position and a closed position, wherein the deformable lip (331) comprises a flexible material and is positioned closer to the first side (311) of the base (310) in the closed position than in the open position.

2. The apparatus of claim 1, further comprising a film layer (300) coupled to the second side (312) of the base (310).

3. The apparatus of claim 1, wherein the post member has a thickness between the first side of the base and the top flange, wherein the winding surface (318) is defined around the thickness (313) of the post member (315) and wherein the deformable lip has a radial length extending transverse to the thickness of the post member.

4. The apparatus of claim 1, wherein the conduit (320) is coiled around the winding surface (318) of the post member (315), in particular wherein the conduit (320) is coiled around the winding surface (318) of the post member (315) in a single layer having a thickness that does not exceed an outside diameter of the conduit (320), and in particular wherein the conduit (320) is coiled around the winding surface (318) such that each revolution of the conduit (320) around the winding surface (318) lies in the same plane.

5. The apparatus of claim 1, wherein the conduit (320) comprises a first end (321) and a second end (322), the first end (321) for fluidly coupling to a negative-pressure source and the second end (322) for fluidly coupling to a tissue site.

6. The apparatus of claim 1, wherein the conduit (320) is a first conduit (320), the apparatus comprising a second conduit (1000) fluidly coupled to the first conduit (320).

7. The apparatus of claim 1, wherein the post member (315) comprises a first post (1200) and a second post (1205) adjacent the first post (1200).

8. The apparatus of claim 7, further comprising:
a longitudinal recess (1220) extending between the first post (1200) and the second post (1205) in a first plane (1232) adjacent the first side (311) of the base (310);
a first taper (1225) extending from the longitudinal recess (1220) and around the first post (1200) to a second plane (1233) above the first plane; and
a second taper (1230) extending from the longitudinal recess (1220) and around the second post (1205) to the second plane (1233);
in particular wherein the conduit (320) is inserted into the longitudinal recess (1220) in the first plane (1232), and in particular wherein:
the conduit (320) extends from the longitudinal recess (1220) into the second plane (1233) via the first taper (1225) or the second taper (1230); and
the conduit (320) is coiled around the first post (1200) and the second post (1205) such that each revolution of the conduit (320) around an exterior surface of the first post (1200) and the second post (1205) lies in the second plane (1233).

9. The apparatus of claim 7, wherein the top flange (330) comprises a first portion (1210) coupled to the first post (1200) and a second portion (1215) coupled to the second post (1205).

10. The apparatus of claim 1, further comprising:
an internal cavity (1300) disposed in the base (310) and the post member (315);
a first port (1305) coupled to a side of the post member (315) and fluidly coupled to the internal cavity (1300); and
a second port (1310) coupled to a side of the base (310) and fluidly coupled to the internal cavity (1300), the second port (1310) opposite the first port (1305);
in particular wherein the conduit (320) is a first conduit (320) fluidly coupled to the first port (1305), the apparatus further comprising a second conduit (1000) fluidly coupled to the second port (1310).

11. The apparatus of claim 1, further comprising:
a port (325) coupled to the post member (315) wherein the port (325) fluidly couples the conduit (320) to a tissue site through an aperture (335) in the second side (312) of the base (310).

12. The apparatus of claim 11, wherein the conduit (320) comprises a first end (321) and a second end (322), the first end (321) fluidly coupled to a negative pressure source and the second end (322) fluidly coupled to the port (325), in particular wherein the post member (315) includes an internal chamber (336) in fluid communication between the port (325) and the aperture (335) such that the conduit (320) is fluidly coupled to the tissue site through the internal chamber (336) and the aperture (335).

13. The apparatus of claim 11, wherein the deformable lip (331) of the top flange (330) extends over the base (310) in the closed position, in particular wherein the deformable lip (331) extends inward toward the first side (311) of the base (310) in the closed position to hold the conduit (320) in place between the deformable lip (331) and the first side (311) of the base (310), and in particular wherein the deformable lip (331) extends outward from the first side (311) of the base (310) in the open position to expose the winding surface (318).

14. The apparatus of claim 11, wherein the port (325) is positioned in a recess (323) in the winding surface (318).

15. A system for treating a tissue site, comprising:
the apparatus of claim 11;
a tissue interface (120) for positioning over the tissue site; and
a cover (125) positioned over the tissue interface to create a sealed space between the cover (125) and the tissue site, wherein the second side (312) of the base (310) is coupled to the cover (125) such that the port (325) is in fluid communication with the tissue interface (120) through the cover (125) and the aperture (335) in the second side (312) of the base (310).

## Patentansprüche

1. Eine Vorrichtung für eine Handhabung von Schläuchen, aufweisend:
eine Basis (310), die eine erste Seite (311) und eine zweite Seite (312) vorweist;
ein Stiftelement (315), das sich von der ersten Seite (311) der Basis (310) erstreckt, wobei das Stiftelement (315) eine Windungsoberfläche (318) aufweist, die eine Leitung (320) trägt; und
einen oberen Flansch (330), der an das Stiftelement (315) gegenüber der Basis (310) gekoppelt ist, wobei der obere Flansch (330) eine verformbare Lippe (331) aufweist, die sich zwischen einer offenen Position und einer geschlossenen Position bewegt, wobei die verformbare Lippe (331) ein flexibles Material aufweist und in der geschlossenen Position näher an der ersten Seite (311) der Basis (310) als in der offenen Position positioniert ist.

2. Die Vorrichtung nach Anspruch 1, ferner aufweisend eine Folienschicht (300), die mit der ersten Seite (312) der Basis (310) gekoppelt ist.

3. Die Vorrichtung nach Anspruch 1, wobei das Stiftelement eine Dicke zwischen der ersten Seite der Basis und dem oberen Flansch vorweist, wobei die Windungsoberfläche (318) um die Dicke (313) des Stiftelements (315) herum definiert ist und wobei die verformbare Lippe eine radiale Länge vorweist, die sich quer zu der Dicke des Stiftelements erstreckt.

4. Die Vorrichtung nach Anspruch 1, wobei die Leitung (320) um die Windungsoberfläche (318) des Stiftelements (315) gewickelt ist, insbesondere wobei die Leitung (320) um die Wickeloberfläche (318) des Stiftelements (315) herum in einer einzigen Schicht gewickelt ist, die eine Dicke vorweist, die einen Außendurchmesser der Leitung (320) nicht überschreitet, und insbesondere wobei die Leitung (320) derart um die Windungsoberfläche (318) gewickelt ist, dass jede Umdrehung der Leitung (320) um die Windungsoberfläche (318) in derselben Ebene liegt.

5. Die Vorrichtung nach Anspruch 1, wobei die Leitung (320) ein erstes Ende (321) und ein zweites Ende (322), das erste Ende (321) zum fluidischen Koppeln mit einer Unterdruckquelle und das zweite Ende (322) zum fluidischen Koppeln mit einer Gewebestelle, aufweist.

6. Die Vorrichtung nach Anspruch 1, wobei die Leitung (320) eine erste Leitung (320) ist, die Vorrichtung aufweisend eine zweite Leitung (1000), die mit der ersten Leitung (320) fluidisch gekoppelt ist.

7. Die Vorrichtung nach Anspruch 1, wobei das Stiftelement (315) einen ersten Stift (1200) und einen zweiten Stift (1205) benachbart zu dem ersten Stift (1200) aufweist.

8. Die Vorrichtung nach Anspruch 7, ferner aufweisend:
eine Längsaussparung (1220), die sich zwischen dem ersten Stift (1200) und dem zweiten Stift (1205) in einer ersten Ebene (1232) benachbart zu der ersten Seite (311) der Basis (310) erstreckt;
eine erste Verjüngung (1225), die sich von der Längsaussparung (1220) und um den ersten Stift (1200) zu einer zweiten Ebene (1233) über der ersten Ebene erstreckt; und
eine zweite Verjüngung (1230), die sich von der Längsaussparung (1220) und um den zweiten Stift (1205) zu der zweiten Ebene (1233) erstreckt;
insbesondere wobei die Leitung (320) in die Längsaussparung (1220) in der ersten Ebene (1232) eingesetzt ist, und insbesondere wobei:
sich die Leitung (320) von der Längsaussparung (1220) über die erste Verjüngung (1225) oder die zweite Verjüngung (1230) in die zweite Ebene (1233) erstreckt; und
die Leitung (320) derart um den ersten Stift (1200) und den zweiten Stift (1205) gewickelt ist, dass jede Umdrehung der Leitung (320) um eine Außenoberfläche des ersten Stifts (1200) und des zweiten Stifts (1205) in der zweiten Ebene (1233) liegt.

9. Die Vorrichtung nach Anspruch 7, wobei der obere Flansch (330) einen ersten Abschnitt (1210), der an den ersten Stift (1200) gekoppelt ist, und einen zweiten Abschnitt (1215), der an den zweiten Stift (1205) gekoppelt ist, aufweist.

10. Die Vorrichtung nach Anspruch 1, ferner aufweisend:
einen inneren Hohlraum (1300), der in der Basis (310) und dem Stiftelement (315) angeordnet ist;
einen ersten Anschluss (1305), der mit einer Seite des Stiftelements (315) gekoppelt ist und mit dem inneren Hohlraum (1300) fluidisch gekoppelt ist; und
einen zweiten Anschluss (1310), der mit einer Seite der Basis (310) gekoppelt ist und mit dem inneren Hohlraum (1300) fluidisch gekoppelt ist, wobei der zweite Anschluss (1310) dem ersten Anschluss (1305) gegenüberliegt;
insbesondere wobei die Leitung (320) eine erste Leitung (320) ist, die mit dem ersten Anschluss (1305) fluidisch gekoppelt ist, die Vorrichtung ferner aufweisend eine zweite Leitung (1000), die mit dem zweiten Anschluss (1310) fluidisch gekoppelt ist.

11. Die Vorrichtung nach Anspruch 1, ferner aufweisend:
einen Anschluss (325), der mit dem Stiftelement (315) gekoppelt ist, wobei der Anschluss (325) die Leitung (320) mit einer Gewebestelle durch eine Öffnung (335) in der zweiten Seite (312) der Basis (310) fluidisch koppelt.

12. Die Vorrichtung nach Anspruch 11, wobei die Leitung (320) ein erstes Ende (321) und ein zweites Ende (322) aufweist, wobei das erste Ende (321) mit einer Unterdruckquelle fluidisch gekoppelt ist und das zweite Ende (322) mit dem Anschluss (325) fluidisch gekoppelt ist, insbesondere wobei das Stiftelement (315) eine innere Kammer (336) in Fluidverbindung zwischen dem Anschluss (325) und der Öffnung (335) aufweist, derart, dass die Leitung (320) durch die innere Kammer (336) und die Öffnung (335) mit der Gewebestelle fluidisch gekoppelt ist.

13. Die Vorrichtung nach Anspruch 11, wobei sich die verformbare Lippe (331) des oberen Flansches (330) über die Basis (310) in der geschlossenen Position erstreckt, insbesondere wobei sich die verformbare Lippe (331) nach innen zu der ersten Seite (311) der Basis (310) hin in der geschlossenen Position erstreckt, um die Leitung (320) zwischen der verformbaren Lippe (331) und der ersten Seite (311) der Basis (310) an Ort und Stelle zu halten, und insbesondere wobei sich die verformbare Lippe (331) nach außen von der ersten Seite (311) der Basis (310) in der offenen Position erstreckt, um die Wickeloberfläche (318) freizulegen.

14. Die Vorrichtung nach Anspruch 11, wobei der Anschluss (325) in einer Aussparung (323) in der Windungsoberfläche (318) positioniert ist.

15. Ein System zum Behandeln einer Gewebestelle, aufweisend:
die Vorrichtung nach Anspruch 11;
eine Gewebeschnittstelle (120) zum Positionierung über der Gewebestelle; und
eine Abdeckung (125), die über der Gewebeschnittstelle positioniert ist, um einen abgedichteten Raum zwischen der Abdeckung (125) und der Gewebestelle zu schaffen, wobei die zweite Seite (312) der Basis (310) mit der Abdeckung (125) gekoppelt ist, derart, dass der Anschluss (325) mit der Gewebeschnittstelle (120) durch die Abdeckung (125) und die Öffnung (335) auf der zweiten Seite (312) der Basis (310) in Fluidverbindung steht.

## Revendications

1. Appareil permettant de gérer une tubulure, comprenant :
une base (310) ayant un premier côté (311) et un second côté (312) ;
un élément montant (315) s'étendant à partir du premier côté (311) de la base (310), l'élément montant (315) comportant une surface d'enroulement (318) qui porte un conduit (320) ; et
une bride supérieure (330) accouplée à l'élément montant (315) à l'opposé de la base (310), la bride supérieure (330) comportant une lèvre déformable (331) qui se déplace entre une position ouverte et une position fermée, dans lequel la lèvre déformable (331) comprend un matériau flexible et est positionnée plus près du premier côté (311) de la base (310) dans la position fermée que dans la position ouverte.

2. Appareil selon la revendication 1, comprenant en outre une couche de film (300) accouplée au second côté (312) de la base (310).

3. Appareil selon la revendication 1, dans lequel l'élément montant a une épaisseur entre le premier côté de la base et la bride supérieure, dans lequel la surface d'enroulement (318) est définie autour de l'épaisseur (313) de l'élément montant (315) et dans lequel la lèvre déformable a une longueur radiale s'étendant transversalement à l'épaisseur de l'élément montant.

4. Appareil selon la revendication 1, dans lequel le conduit (320) est enroulé autour de la surface d'enroulement (318) de l'élément montant (315), en particulier dans lequel le conduit (320) est enroulé autour de la surface d'enroulement (318) de l'élément montant (315) en une couche unique ayant une épaisseur qui ne dépasse pas un diamètre extérieur du conduit (320), et en particulier dans lequel le conduit (320) est enroulé autour de la surface d'enroulement (318) de telle sorte que chaque révolution du conduit (320) autour de la surface d'enroulement (318) se trouve dans le même plan.

5. Appareil selon la revendication 1, dans lequel le conduit (320) comprend une première extrémité (321) et une seconde extrémité (322), la première extrémité (321) pour l'accouplement fluidique à une source de pression négative et la seconde extrémité (322) pour l'accouplement fluidique à un site tissulaire.

6. Appareil selon la revendication 1, dans lequel le conduit (320) est un premier conduit (320), l'appareil comprenant un second conduit (1000) accouplé fluidiquement au premier conduit (320).

7. Appareil selon la revendication 1, dans lequel l'élément montant (315) comprend un premier montant (1200) et un second montant (1205) adjacent au premier montant (1200).

8. Appareil selon la revendication 7, comprenant en outre :
un évidement longitudinal (1220) s'étendant entre le premier montant (1200) et le second montant (1205) dans un premier plan (1232) adjacent au premier côté (311) de la base (310) ;
un premier rétrécissement (1225) s'étendant à partir de l'évidement longitudinal (1220) et autour du premier montant (1200) jusqu'à un second plan (1233) au-dessus du premier plan ; et
un second rétrécissement (1230) s'étendant à partir de l'évidement longitudinal (1220) et autour du second montant (1205) jusqu'au second plan (1233) ;
en particulier dans lequel le conduit (320) est inséré dans l'évidement longitudinal (1220) dans le premier plan (1232), et en particulier dans lequel :
le conduit (320) s'étend à partir de l'évidement longitudinal (1220) dans le second plan (1233) par l'intermédiaire du premier rétrécissement (1225) ou du second rétrécissement (1230) ; et
le conduit (320) est enroulé autour du premier montant (1200) et du second montant (1205) de telle sorte que chaque révolution du conduit (320) autour d'une surface extérieure du premier montant (1200) et du second montant (1205) se trouve dans le second plan (1233).

9. Appareil selon la revendication 7, dans lequel la bride supérieure (330) comprend une première partie (1210) accouplée au premier montant (1200) et une seconde partie (1215) accouplée au second montant (1205).

10. Appareil selon la revendication 1, comprenant en outre :
une cavité interne (1300) disposée dans la base (310) et l'élément montant (315) ;
un premier orifice (1305) accouplé à un côté de l'élément montant (315) et accouplé fluidiquement à la cavité interne (1300) ; et
un second orifice (1310) accouplé à un côté de la base (310) et accouplé fluidiquement à la cavité interne (1300), le second orifice (1310) étant opposé au premier orifice (1305) ;
en particulier dans lequel le conduit (320) est un premier conduit (320) accouplé fluidiquement au premier orifice (1305), l'appareil comprenant en outre un second conduit (1000) accouplé fluidiquement au second orifice (1310).

11. Appareil selon la revendication 1, comprenant en outre :
un orifice (325) accouplé à l'élément montant (315), dans lequel l'orifice (325) accouple fluidiquement le conduit (320) à un site tissulaire à travers une ouverture (335) dans le second côté (312) de la base (310).

12. Appareil selon la revendication 11, dans lequel le conduit (320) comprend une première extrémité (321) et une seconde extrémité (322), la première extrémité (321) étant accouplée fluidiquement à une source de pression négative et la seconde extrémité (322) accouplée fluidiquement à l'orifice (325), en particulier dans lequel l'élément montant (315) comporte une chambre interne (336) en communication fluidique entre l'orifice (325) et l'ouverture (335) de telle sorte que le conduit (320) est accouplé fluidiquement au site tissulaire à travers la chambre interne (336) et l'ouverture (335).

13. Appareil selon la revendication 11, dans lequel la lèvre déformable (331) de la bride supérieure (330) s'étend au-dessus de la base (310) dans la position fermée, en particulier dans lequel la lèvre déformable (331) s'étend vers l'intérieur vers le premier côté (311) de la base (310) dans la position fermée pour maintenir le conduit (320) en place entre la lèvre déformable (331) et le premier côté (311) de la base (310), et en particulier dans lequel la lèvre déformable (331) s'étend vers l'extérieur à partir du premier côté (311) de la base (310) dans la position ouverte pour exposer la surface d'enroulement (318).

14. Appareil selon la revendication 11, dans lequel l'orifice (325) est positionné dans un évidement (323) dans la surface d'enroulement (318).

15. Système permettant de traiter un site tissulaire, comprenant :
l'appareil selon la revendication 11 ;
une interface tissulaire (120) destinée à être positionnée au-dessus du site tissulaire ; et
un élément de recouvrement (125) positionné au-dessus de l'interface tissulaire pour créer un espace scellé entre l'élément de recouvrement (125) et le site tissulaire, dans lequel le second côté (312) de la base (310) est accouplé à l'élément de recouvrement (125) de telle sorte que l'orifice (325) est en communication fluidique avec l'interface tissulaire (120) à travers l'élément de recouvrement (125) et l'ouverture (335) dans le second côté (312) de la base (310).
